(19) Europäisches Patentamt
European Patent Office
Office européen des brevets

(11) Numéro de publication: 0 304 353 B1

(12) **FASCICULE DE BREVET EUROPEEN**

(45) Date de publication de fascicule du brevet: **08.09.93**

(51) Int. Cl.[5]: **C07D 401/04**, A61K 31/415

(21) Numéro de dépôt: **88401866.4**

(22) Date de dépôt: **20.07.88**

Le dossier contient des informations techniques présentées postérieurement au dépôt de la demande et ne figurant pas dans le présent fascicule.

(54) **Dérivés de benzimidazole et leur préparation.**

(30) Priorité: **23.07.87 FR 8710409**

(43) Date de publication de la demande: **22.02.89 Bulletin 89/08**

(45) Mention de la délivrance du brevet: **08.09.93 Bulletin 93/36**

(84) Etats contractants désignés: **AT BE CH DE ES FR GB GR IT LI LU NL SE**

(56) Documents cités:
**EP-A- 0 005 318**
**EP-A- 0 145 037**
**EP-A- 0 217 700**

(73) Titulaire: **SYNTHELABO**
**22, Avenue Galilée**
**F-92350 Le Plessis Robinson(FR)**

(72) Inventeur: **Manoury, Philippe**
**L'Orée de Verrières / 38, Av. des Vaupépins**
**F-91370 Verrières le Buisson(FR)**
Inventeur: **Rossey, Guy**
**8, Square Lebrun Voisins le Bretonneux**
**F-78180 Montigny le Bretonneux(FR)**
Inventeur: **Binet, Jean**
**12, Hameau de la Gondole**
**F-91650 Breuillet(FR)**
Inventeur: **Defosse, Gérard**
**29, Rue de Tolbiac**
**F-75013 Paris(FR)**
Inventeur: **Jabri, Najib**
**8, Rue Carle Hebert**
**F-92400 Courbevoie(FR)**

(74) Mandataire: **Thouret-Lemaitre, Elisabeth et al**
**SYNTHELABO, Service Brevets, 22, avenue Galilée, B.P. 72**
**F-92352 Le Plessis-Robinson Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 304 353 B1

## Description

La présente invention a pour objet des dérivés de benzimidazole et leur préparation.

Les composés de l'invention répondent à la formule (I) donnée en annexe dans laquelle
$R_1$ représente un atome d'hydrogène ou le radical méthyle,
$R_2$ représente un atome d'hydrogène, le radical méthyle ou le radical acétyle,
$R_3$ représente un atome d'hydrogène, le radical méthyle ou le radical acétyle,
$R_4$ est un atome d'hydrogène ou d'halogène.

Les sels d'addition que peuvent former les composés (I) avec des acides pharmaceutiquement acceptables font partie de l'invention.
Lorsque $R_1$ et/ou $R_2$ et/ou $R_3$ est un atome d'hydrogène, les formes tautomères des composés font partie de l'invention. $R_2$ et $R_3$ peuvent être considérés comme équivalents lorsque $R_1$ est H.

Les composés (I) sont des intermédiaires utiles pour la préparation des composés finals de la demande de brevet EP 0217700. Ces derniers sont en effet obtenus par cyclisation classique à l'aide d'un alcoxyacrylate d'alkyle, des composés (I) de l'invention. Les composés décrits dans la présente demande permettent, par un procédé avantageux par rapport à celui décrit dans la demande de brevet EP 0217700, d'obtenir les composés revendiqués dans cette dernière demande.
En effet le procédé de préparation de ces composés finals, tel que décrit dans la demande de brevet EP 0217700, nécessitait une réaction à haute température sans solvant, difficile à mettre en oeuvre à l'échelle industrielle. Le procédé de préparation de ces composés finals par cyclisation , qui utilise les composés de la présente invention comme intermédiaires, peut être, lui, réalisé à l'échelle industrielle.

Les composés (I) peuvent être préparés selon le schéma réactionnel donné en annexe :

1. Soit on fait réagir un composé (II) avec le cyanamide, ce qui conduit au composé (I)a dans lequel $R_1$, $R_2$ et $R_3$ sont chacun un atome d'hydrogène,
puis, si on le désire, on fait réagir ce composé (I)a avec de l'anhydride acétique pour obtenir les composés (I)b dans lesquels $R_1$ est H, $R_2$ est $COCH_3$ et $R_3$ est H ou $COCH_3$,
2. Soit on fait réagir un composé (II) d'abord avec le thiocyanate de potassium ou l'isothiocyanate de méthyle, puis avec de l'iodure de méthyle pour obtenir le composé (III) que l'on fait réagir soit avec de l'ammoniac soit avec la méthylamine, soit avec la diméthylamine pour obtenir les composés (I)a dans lesquels $R_1$ est H ou $CH_3$, $R_2$ est H ou $CH_3$ et $R_3$ est H ou $CH_3$, puis, si on le désire, on fait réagir l'un de ces composés (I)a, avec de l'anhydride acétique pour obtenir les composés (I)b dans lesquels soit $R_1$ est $CH_3$, $R_2$ est $COCH_3$ et $R_3$ est H, $CH_3$ ou $COCH_3$, soit $R_1$ est $CH_3$, $R_2$ est H ou $CH_3$ et $R_3$ est $COCH_3$, soit $R_1$ est H, l'un des radicaux $R_2$ et $R_3$ est $CH_3$ et l'autre est $COCH_3$.

La préparation des composés (II) est décrite dans la demande de brevet EP 0217700.

Les exemples suivants illustrent l'invention.
Les spectres IR et RMN confirment la structure des composés.

Exemple 1. N-[[[(fluoro-4 phényl)méthyl]-1 1H-benzimidazolyl-2]-1pipéridinyl-4] N-méthyl-guanidine.

On chauffe à 135 °C pendant 1,5 heure un mélange de 10,1 g (0,03 mode) de [[(fluoro-4 phényl)-méthyl]-1 1H-benzimidazolyl-2]-1 N-méthyl-pipéridinamine-4 avec 1,4 g (0,032 mole) de cyanamide et 1,8 g (0,03 mole) d'acide acétique. On refroidit le mélange puis le reprend avec du chlorure de méthylène au reflux, on filtre le précipité puis le sèche. On obtient ainsi l'acétate de N-[[[(fluoro-4 phényl)méthyl]-1 1H-benzimidazolyl-2]-1 pipéridinyl-4] N-méthyl-guanidine. F = 233 °C (propanol).

Exemple 2. N-[[[(fluoro-4 phényl)méthyl]-1 1H-benzimidazolyl-2]-1 pipéridinyl-4] N-méthyl, N'-acétyl guanidine.

A une suspension de 2 g (0,004 mole) du produit obtenu précédemment dans 30 ml de chlorure de méthylène on ajoute 0,85 ml de méthylate de sodium 5,3 N puis on évapore à sec, on reprend le résidu exempt de méthanol, avec 30 ml de chlorure de méthylène et refroidit le mélange à 5 °C environ. On ajoute à cette tempérautre 0,43 ml d'anhydride acétique en solution dans 5 ml de chlorure de méthylène puis agite pendant 2 h en maintenant la température à 5 °C.
On neutralise avec du méthylate de sodium et purifie le produit par chromatographie sur une colonne de silice (éluant chlorure de méthylène-méthanol 9/1).
On obtient ainsi la N-[[[(fluoro-4 phényl)méthyl]-1 1H-benzimidazolyl-2]-1 pipéridinyl-4] N-méthyl N'-acétyl-guanidine. F = 192 °C.

Exemple 3. N-[[[(fluoro-4 phényl)méthyl]-1 1H benzimidazolyl-2]-1pipéridinyl-4] N,N'diméthyl-guanidine.

3.1. N-[[[(fluoro-4 phényl)méthyl]-1 1H-benzimidazolyl-2]-1pipéridinyl-4] N,N'-diméthyl-thiourée.

On chauffe au reflux, pendant 3 h, 6,8 g (0,02 mole) de [[(fluoro-4 phényl)méthyl]-1 1H-benzimidazolyl-2] N-méthyl pipéridinamine-4 avec 1,6 g (0,022 mole) d'isothiocyanate de méthyle dans 60 ml d'éthanol. On évapore à sec et reprend le résidu avec 50 ml de toluène bouillant. Par refroidissement on obtient la N-[[[(fluoro-4 phényl)méthyl]-1 1H benzimidazolyl-2]-1 pipéridinyl-4] N,N'-diméthyl-thiourée. F = 176 ° C.

3.2. N-[[[(fluoro-4 phényl)méthyl]-1 1H-benzimidazolyl-2]-1pipéridinyl-4] N,N'-diméthyl-guanidine.

On dissout le produit précédent dans 15 ml d'acétonitrile et 20 ml de chlorure de méthylène. On ajoute 0,6 ml d'iodure de méthyle et laisse au repos la nuit. On évapore la solution à sec et obtient une huile que l'on dissout dans 50 ml d une solution saturée d'éthanol ammoniacal. On chauffe la solution à 110 ° C pendant 5 heures dans un autoclave. On évapore à sec, reprend le résidu avec du chlorure de méthylène, séche avec $MgSO_4$, filtre, évapore.
On prépare le fumarate du composé dans un mélange éthanol/éther.
F = 188 ° C (propanol).

Exemple 4. N-[[[(fluoro-4 phényl)méthyl]-1 1H-benzimidazolyl-2]-1pipéridinyl-4] N,N'-diméthyl N''-acétyl-guanidine.

A une solution de 2 g (0,005 mole) de N-[[[(fluoro-4 phényl)-méthyl]-1 1H-benzimidazolyl-2]-1 pipéridinyl-4] N,N'-diméthyl-guanidine dans 20 ml de chlorure de méthylène, refroidie à 5 ° C, on ajoute 0,48 ml d'anhydride acétique en solution dans 5 ml de chlorure de méthylène. On maintient l'agitation pendant 2 h, puis verse le mélange dans de la soude concentrée. On décante, sèche la phase organique avec $MgSO_4$, filtre, évapore. Par addition d'acétone le produit cristallise. On purifie celui-ci par chromatographie sur colonne de silice (éluant chlorure de méthylène méthanol 9/1). On obtient la N-[[[(fluoro-4 phényl)méthyl]-1 1H-benzimidazolyl-2]-1 pipéridinyl-4] N,N'-diméthyl N''-acétyl-guanidine. F = 203 ° C.

Exemple 5. N-[[[(fluoro-4 phényl)méthyl]-1 1H-benzimidazolyl-2]-1pipéridinyl-4] N,N'-diméthyl N',N''-diacétyl-guanidine.

Le filtrat de cristallisation précédent est évaporé à sec, et le résidu obtenu est purifié sur une colonne chromatographique.
On obtient la N-[[[(fluoro-4 phényl)méthyl]-1 1H-benzimidazolyl-2]-1 pipéridinyl-4] N,N'-diméthyl N',N''-diacétyl-guanidine. F = 176 ° C.

Les composés de l'invention préparés à titre d'exemples sont représentés dans le tableau suivant :

Tableau

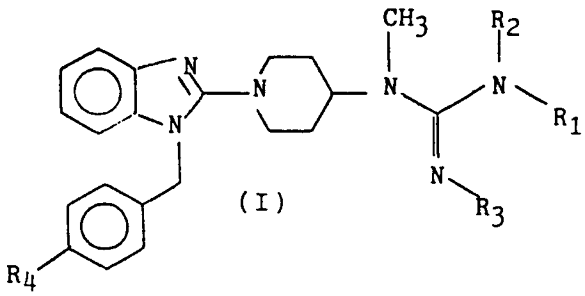

(I)

| Composé | $R_1$ | $R_2$ | $R_3$ | $R_4$ | F(°C) | sel |
|---|---|---|---|---|---|---|
| 1 | H | $CH_3$ | H | F | 188 | fumarate |
| 2 | H | $CH_3$ | $CH_3$ | F | 235 | fumarate |
| 3 | $CH_3CO$ | $CH_3$ | $CH_3$-CO | F | 176 | - |
| 4 | H | $CH_3$ | $CH_3$-CO | F | 203 | - |
| 5 | $CH_3$-CO | H | H | F | 192 | - |
| 6 | H | H | H | F | 233 | acétate |

Les composés de l'invention sont des intermédiaires de synthèse qui permettent de préparer les dérivés de benzimidazole décrits par la Demanderesse dans son brevet EP 0217700, par cyclisation classique à l'aide d'un alcoxyacrylate d'alkyle.

Annexe

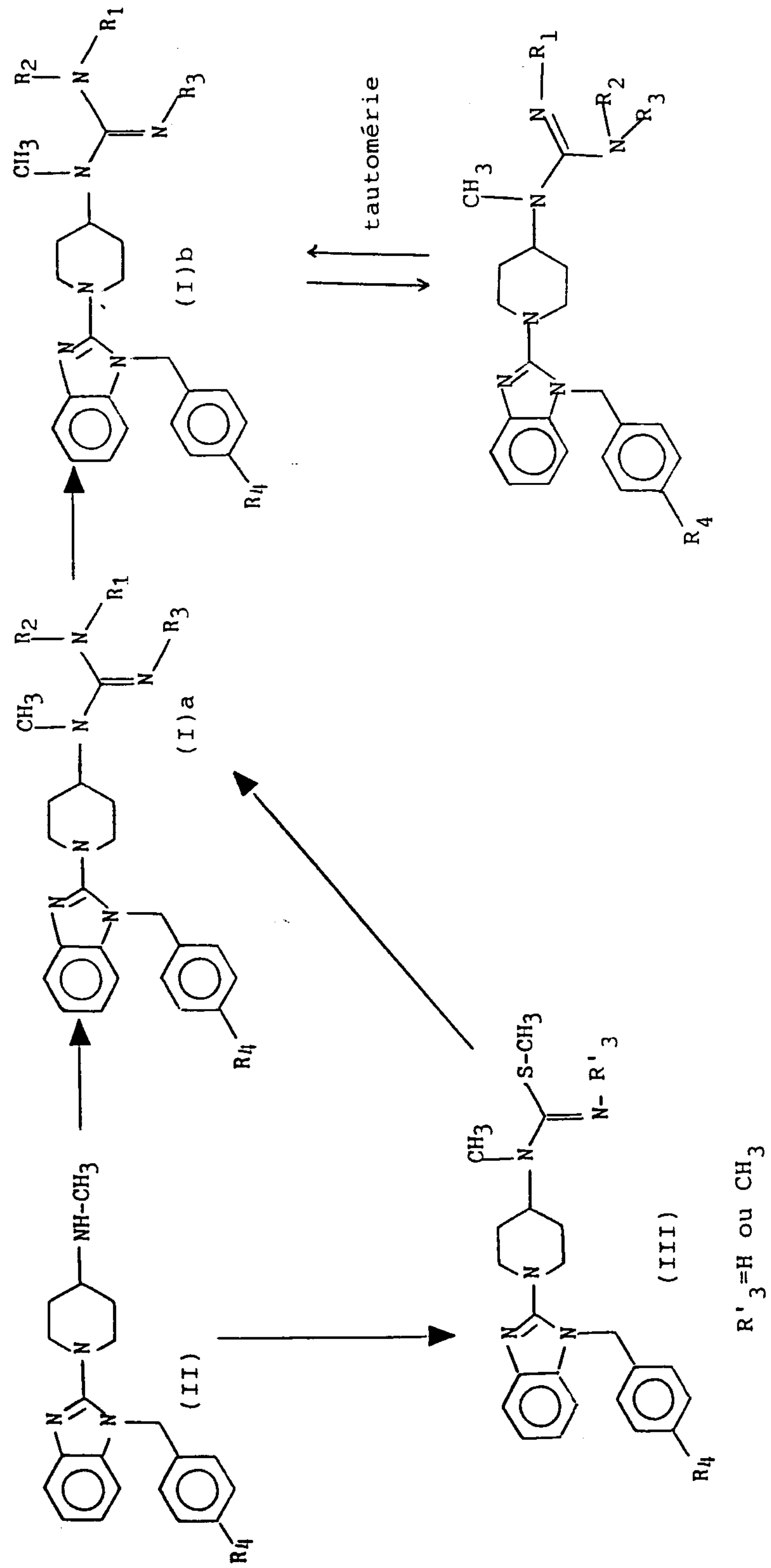


(I)b

(I)a

(II)

(III)

R'3=H ou $CH_3$

## Revendications

**Revendications pour les Etats contractants suivants : AT, BE, CH, DE, FR, GB, IT, LI, LU, NL, SE**

**1.** Dérivés de benzimidazole répondant à la formule (I),

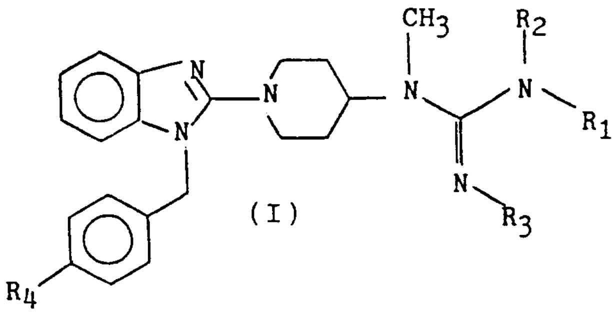

(I)

dans laquelle
$R_1$ représente un atome d'hydrogène ou le radical méthyle,
$R_2$ représente un atome d'hydrogène, le radical méthyle ou le radical acétyle,
$R_3$ représente un atome d'hydrogène, le radical méthyle ou le radical acétyle,
$R_4$ est un atome d'hydrogène ou d'halogène,
ainsi que leurs sels d'addition aux acides pharmaceutiquement acceptables, et les formes tautomères des composés.

**2.** Procédé de préparation des composés selon la revendication 1, procédé caractérisé en ce que
1. soit on fait réagir un composé (II)

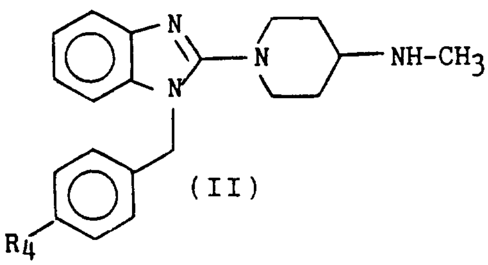

(II)

avec le cyanamide, ce qui conduit au composé (I)a

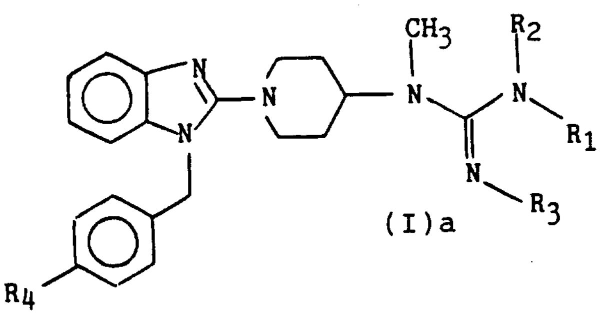

(I)a

dans lequel $R_1$, $R_2$ et $R_3$ sont chacun un atome d'hydrogène, puis si on le désire, on fait réagir ce composé (I)a, avec de l'anhydride acétique pour obtenir les composés (I)b

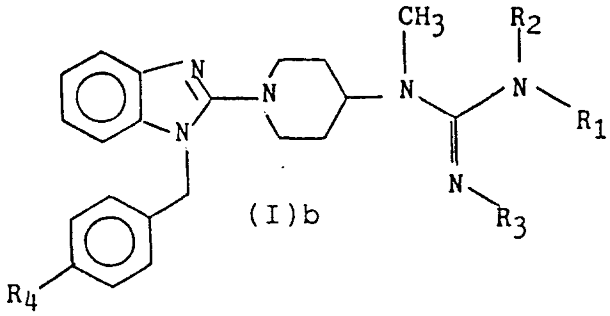

(I)b

dans lesquels $R_1$ est H, $R_2$ est $COCH_3$ et $R_3$ est H ou $COCH_3$,
2. soit on fait réagir un composé (II)

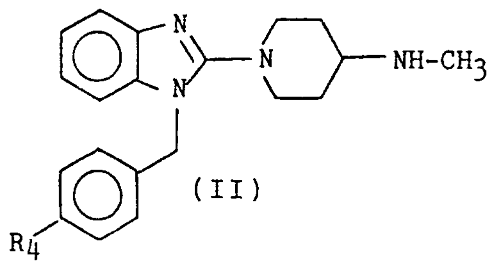

(II)

d'abord avec du thiocyanate de potassium ou avec de l'isothiocyanate de méthyle, puis avec de l'iodure de méthyle pour obtenir le composé (III)

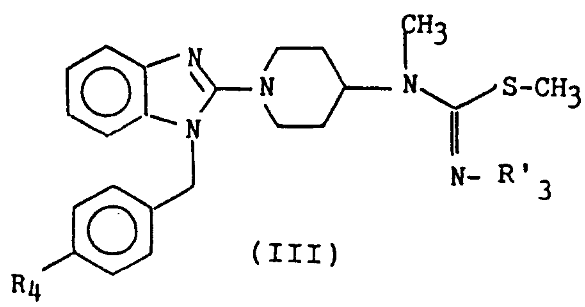

(III)

$R'_3$=H ou $CH_3$

que l'on fait réagir soit avec de l'ammoniac soit avec la méthylamine soit avec la diméthylamine pour obtenir les composés (I)a

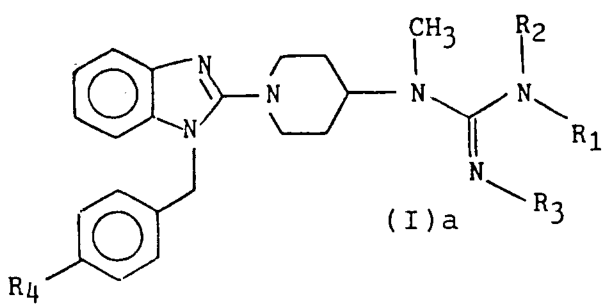

(I)a

dans lesquels $R_1$ est H ou $CH_3$, $R_2$ est H ou $CH_3$ et $R_3$ est H ou $CH_3$,
puis si on le désire, on fait réagir l'un de ces composés (I)a, avec de l'anhydride acétique pour

obtenir les composés (I)b

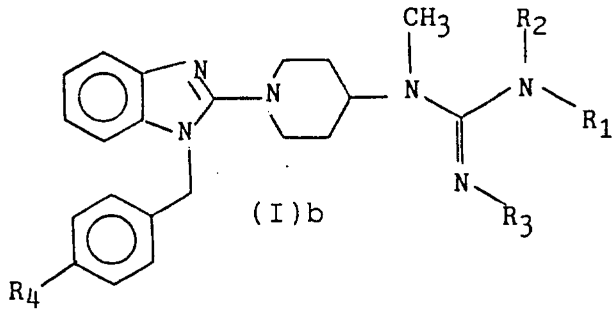

(I)b

dans lesquels soit $R_1$ est $CH_3$, $R_2$ est $COCH_3$ et $R_3$ est H, $CH_3$ ou $COCH_3$, soit $R_1$ est $CH_3$, $R_2$ est H ou $CH_3$ et $R_3$ est $COCH_3$, soit $R_1$ est H, l'un des radicaux $R_2$ et $R_3$ est $CH_3$ et l'autre est $COCH_3$

**3.** Application des composés de la revendication 1 comme intermédiaires de synthèse pour la préparation des composés des dérivés de benzimidazole répondant à la formule (I)

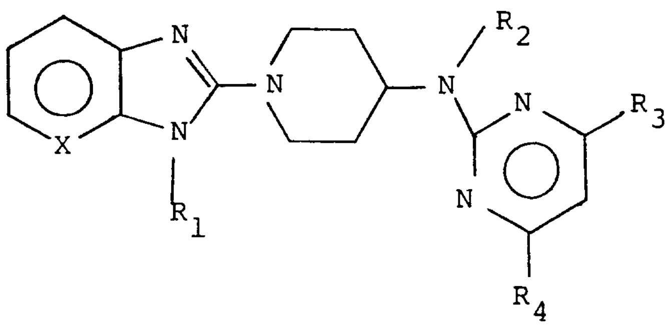

(I)

dans laquelle
X est CH ou N,
$R_1$ est soit un atome d'hydrogène, soit un radical benzyle pouvant porter de 1 à 3 substituants choisis parmi les atomes d'halogène, les radicaux trifluorométhyle, $(C_{1-4})$alkyles, $(C_{1-4})$alcoxy, cyano, méthylthio, méthylsulfinyle et méthylsulfonyle, soit un radical hétérocycle-méthyle dans lequel l'hétérocycle peut être un radical pyridinyle, thiényle ou furannyle pouvant porter un ou plusieurs substituants, $R_2$ est un atome d'hydrogène ou un radical $(C_{1-4})$alkyle, $R_3$ est un atome d'hydrogène ou le radical hydroxy, $R_4$ est un atome d'hydrogène ou un radical $(C_{1-4})$alkyle, éventuellement sous forme de tautomères lorsque $R_3$ est OH, ainsi que leurs sels d'addition aux acides pharmaceutiquemnet acceptables.

**Revendications pour les Etats contractants suivants : ES, GR**

**1.** Procédé de préparation de dérivés de benzimidazole répondant à la formule (I),

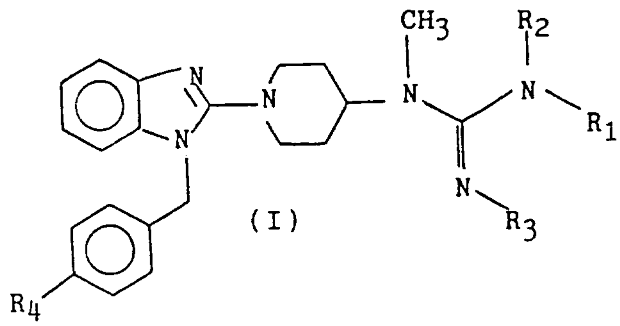

(I)

dans laquelle
$R_1$ représente un atome d'hydrogène ou le radical méthyle,
$R_2$ représente un atome d'hydrogène, le radical méthyle ou le radical acétyle,
$R_3$ représente un atome d'hydrogène, le radical méthyle ou le radical acétyle,
$R_4$ est un atome d'hydrogène ou d'halogène,
ainsi que de leurs sels d'addition aux acides pharmaceutiquement acceptables, et des formes tautomères des composés, procédé caractérisé en ce que

1. soit on fait réagir un composé (II)

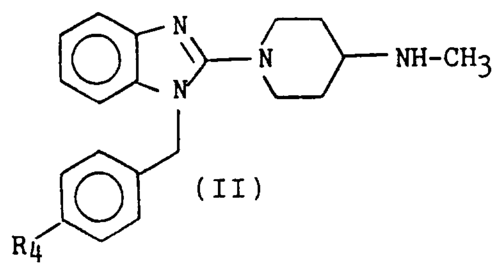

(II)

avec le cyanamide, ce qui conduit au composé (I)a

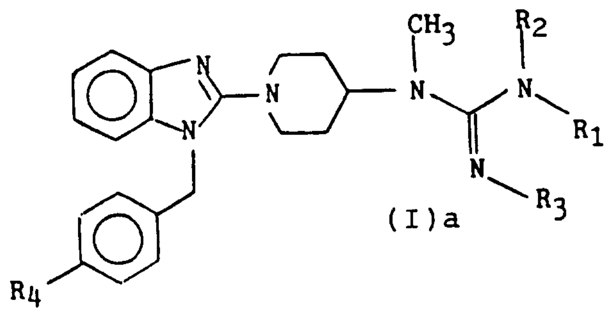

(I)a

dans lequel $R_1$, $R_2$ et $R_3$ sont chacun un atome d'hydrogène, puis si on le désire, on fait réagir ce composé (I)a, avec de l'anhydride acétique pour obtenir les composés (I)b

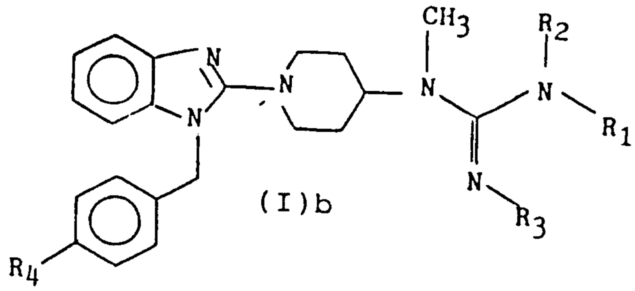

(I)b

dans lesquels $R_1$ est H, $R_2$ est $COCH_3$ et $R_3$ est H ou $COCH_3$,
2. soit on fait réagir un composé (II)

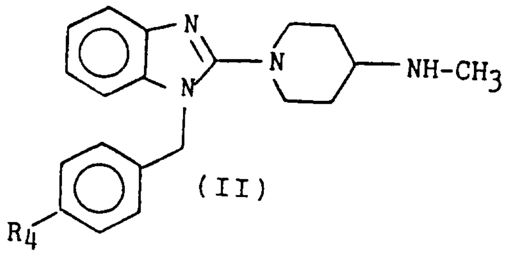

(II)

d'abord avec du thiocyanate de potassium ou avec de l'isothiocyanate de méthyle, puis avec de l'iodure de méthyle pour obtenir le composé (III)

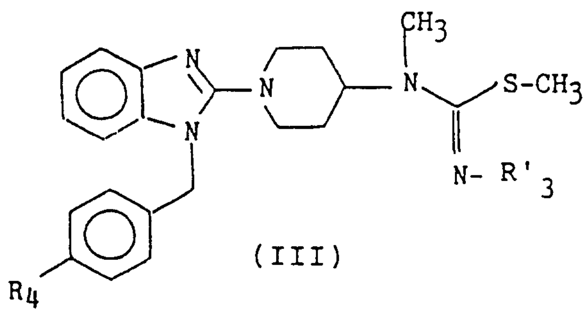

(III)

$R'_3$=H ou $CH_3$

que l'on fait réagir soit avec de l'ammoniac soit avec la méthylamine soit avec la diméthylamine pour obtenir les composés (I)a

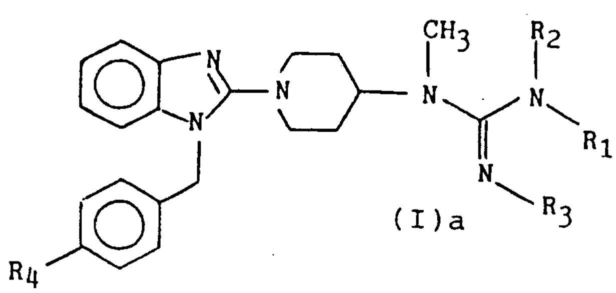

(I)a

dans lesquels $R_1$ est H ou $CH_3$, $R_2$ est H ou $CH_3$ et $R_3$ est H ou $CH_3$, puis si on le désire, on fait réagir l'un de ces composés (I)a, avec de l'anhydride acétique pour obtenir les composés (I)b

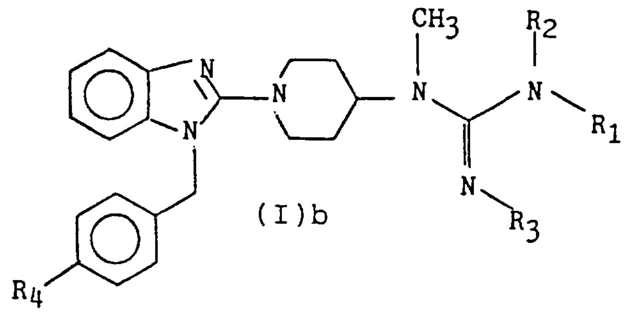

(I)b

dans lesquels soit $R_1$ est $CH_3$, $R_2$ est $COCH_3$ et $R_3$ est H, $CH_3$ ou $COCH_3$, soit $R_1$ est $CH_3$, $R_2$ est H ou $CH_3$ et $R_3$ est $COCH_3$, soit $R_1$ est H, l'un des radicaux $R_2$ et $R_3$ est $CH_3$ et l'autre est $COCH_3$.

## Claims

**Claims for the following Contracting States: AT, BE, CH, DE, FR, GB, IT, LI, LU, NL, SE**

**1.** Benzimidazole derivatives corresponding to formula (I)

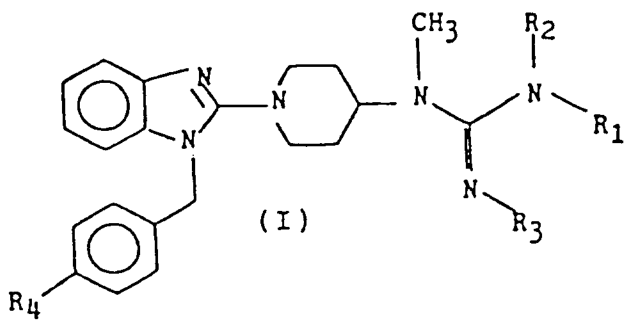

(I)

in which
$R_1$ denotes a hydrogen atom or the methyl radical,
$R_2$ denotes a hydrogen atom, the methyl radical or the acetyl radical,
$R_3$ denotes a hydrogen atom, the methyl radical or the acetyl radical, and
$R_4$ is a hydrogen or halogen atom,
and their addition salts with pharmaceutically acceptable acids, and the tautomeric forms of the compounds.

**2.** Process for the preparation of the compounds according to Claim 1, which process is characterised in that

1. either a compound (II)

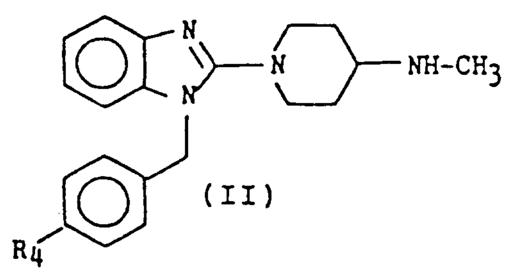

(II)

is reacted
with cyanamide, which results in the compound (I)a

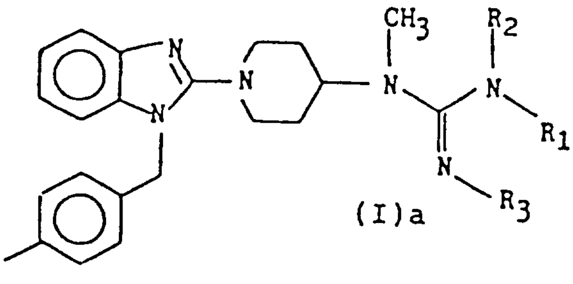

(I)a

in which each of $R_1$, $R_2$ and $R_3$ is a hydrogen atom, and, if desired, this compound (I)a is then reacted with acetic anhydride to obtain the compounds (I)b

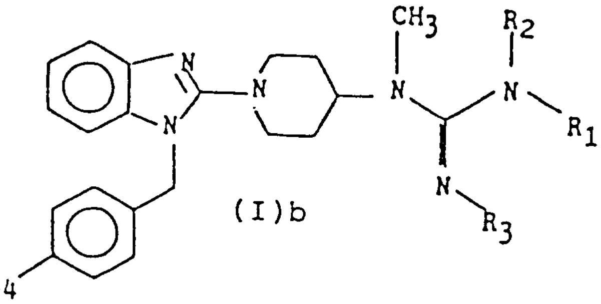

(I)b

in which $R_1$ is H, $R_2$ is $COCH_3$ and $R_3$ is H or $COCH_3$,
2. or a compound (II)

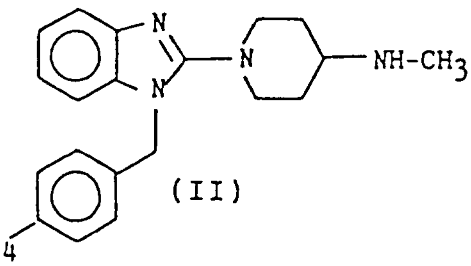

(II)

is first reacted with potassium thiocyanate or with methyl isothiocyanate, and then with methyl iodide to obtain the compound (III)

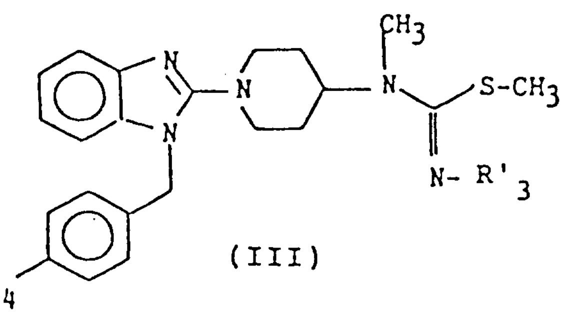

(III)

$R'_3$=H or $CH_3$

which is reacted either with ammonia or with methylamine or with dimethylamine to obtain the compounds (I)a

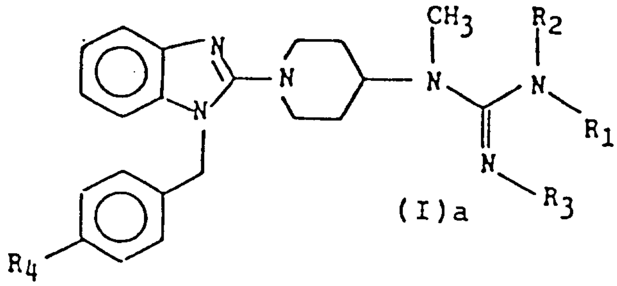

(I)a

in which $R_1$ is H or $CH_3$, $R_2$ is H or $CH_3$ and $R_3$ is H or $CH_3$, then, if desired, one of these compounds (I)a is reacted with acetic anhydride to obtain the compounds (I)b

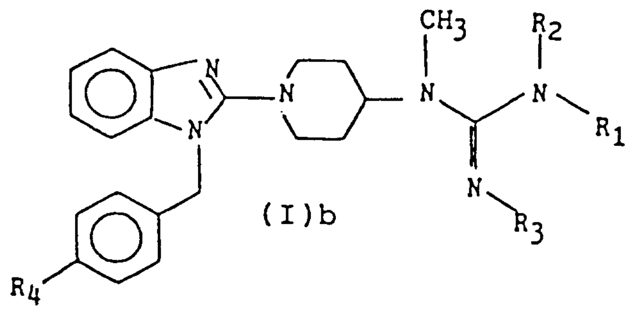

(I)b

in which either: $R_1$ is $CH_3$, $R_2$ is $COCH_3$ and $R_3$ is H, $CH_3$ or $COCH_3$, or: $R_1$ is $CH_3$, $R_2$ is H or $CH_3$ and $R_3$ is $COCH_3$, or: $R_1$ is H, one of the radicals $R_2$ and $R_3$ is $CH_3$ and the other is $COCH_3$.

**3.** Application of the compounds of Claim 1 as synthesis intermediates for the preparation of the compounds of the benzimidazole derivatives corresponding to formula

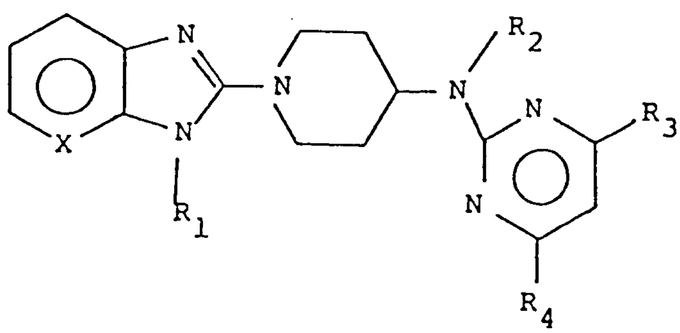

in which
X is CH or N,
$R_1$ is either a hydrogen atom or: a benzyl radical capable of bearing from 1 to 3 substituents chosen from halogen atoms and trifluoromethyl, $C_{1-4}$ alkyl, $C_{1-4}$alkoxy, cyano, methylthio, methylsulphinyl and methylsulphonyl radicals, or a heterocyclomethyl radical in which the heterocyclic ring may be a pyridyl, thienyl or furyl radical capable of bearing one or more substituents,
$R_2$ is a hydrogen atom or a $C_{1-4}$ alkyl radical,
$R_3$ is a hydrogen atom or the hydroxyl radical,
$R_4$ is a hydrogen atom or a $C_{1-4}$ alkyl radical,
optionally in the form of tautomers when $R_3$ is OH, and their salts of addition to pharmaceutically acceptable acids.

**Claims for the following Contracting States: ES, GR**

**1.** Process for the preparation of benzimidazole derivatives corresponding to formula (I)

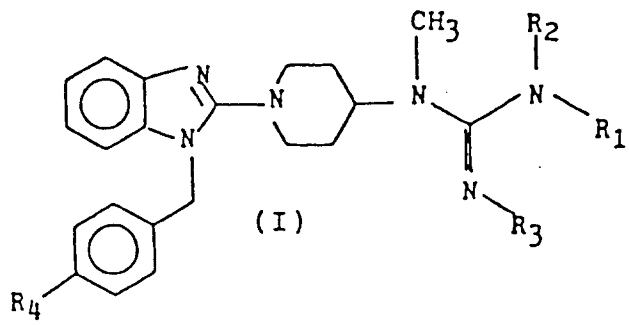

in which
$R_1$ denotes a hydrogen atom or the methyl radical,
$R_2$ denotes a hydrogen atom, the methyl radical or the acetyl radical,
$R_3$ denotes a hydrogen atom, the methyl radical or the acetyl radical, and
$R_4$ is a hydrogen or halogen atom,
and their addition salts with pharmaceutically acceptable acids, and the tautomeric forms of the compounds, which process is characterised in that

1. either a compound (II)

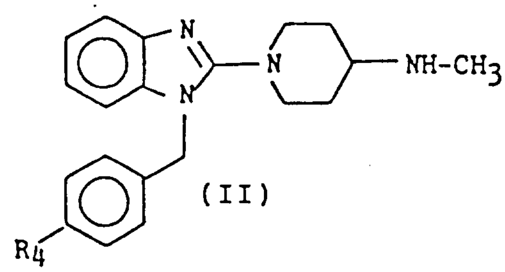

is reacted
with cyanamide, which results in the compound (I)a

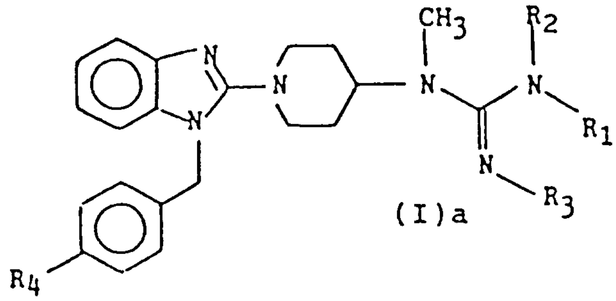

in which each of $R_1$, $R_2$ and $R_3$ is a hydrogen atom, and, if desired, this compound (I)a is then reacted with acetic anhydride to obtain the compounds (I)b

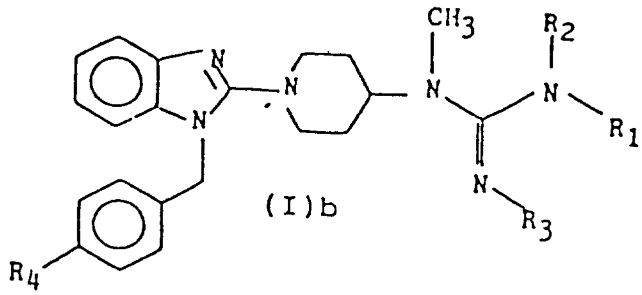

(I)b

in which $R_1$ is H, $R_2$ is $COCH_3$ and $R_3$ is H or $COCH_3$,

2. or a compound (II)

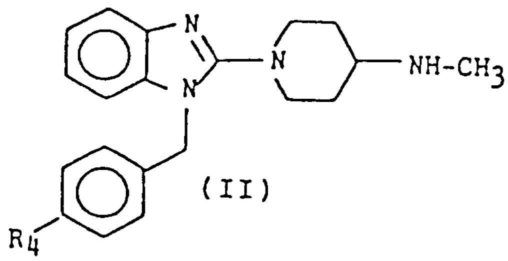

(II)

is first reacted with potassium thiocyanate or with methyl isothiocyanate, and then with methyl iodide to obtain the compound (III)

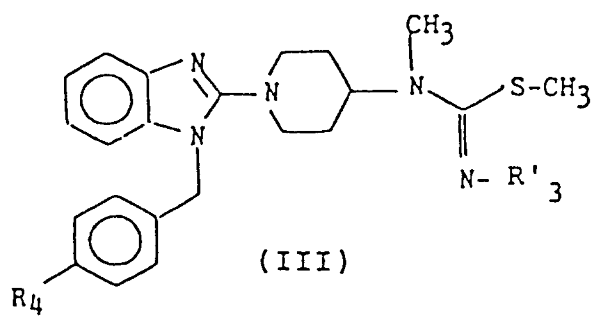

(III)

$R'_3$=H or $CH_3$

which is reacted either with ammonia or with methylamine or with dimethylamine to obtain the compounds (I)a

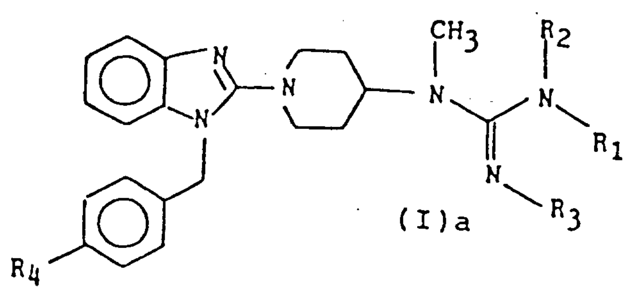

(I)a

in which $R_1$ is H or $CH_3$, $R_2$ is H or $CH_3$ and $R_3$ is H or $CH_3$, then, if desired, one of these compounds (I)a is reacted with acetic anhydride to obtain the compounds (I)b

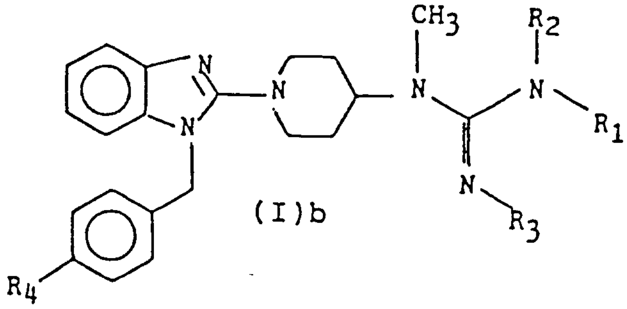

(I)b

in which either: $R_1$ is $CH_3$, $R_2$ is $COCH_3$ and $R_3$ is H, $CH_3$ or $COCH_3$, or: $R_1$ is $CH_3$, $R_2$ is H or $CH_3$ and $R_3$ is $COCH_3$, or: $R_1$ is H, one of the radicals $R_2$ and $R_3$ is $CH_3$ and the other is $COCH_3$.

**Patentansprüche**
**Patentansprüche für folgende Vertragsstaaten : AT, BE, CH, DE, FR, GB, IT, LI, LU, NL, SE**

**1.** Benzimidazol-Derivate der Formel (I)

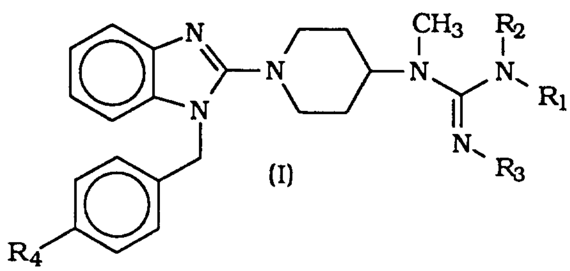

(I)

in der
$R_1$ ein Wasserstoffatom oder eine Methylgruppe.
$R_2$ ein Wasserstoffatom, eine Methylgruppe oder eine Acetylgruppe,
$R_3$ ein Wasserstoffatom, eine Methylgruppe oder eine Acetylgruppe,
$R_4$ ein Wasserstoffatom oder ein Halogenatom
bedeuten sowie deren Additionssalze mit pharmazeutisch annehmbaren Säuren, sowie die tautomeren Formen dieser Verbindungen.

**2.** Verfahren zur Herstellung der Verbindungen nach Anspruch 1, **dadurch gekennzeichnet,** daß man
1. entweder eine Verbindung (II)

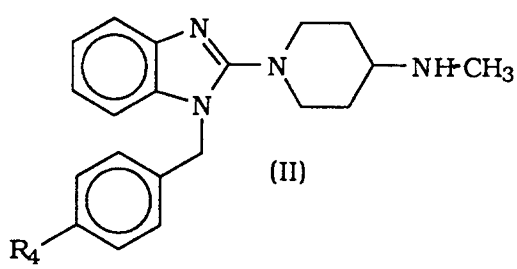

(II)

mit Cyanamid umsetzt, so daß man die Verbindung (I)a

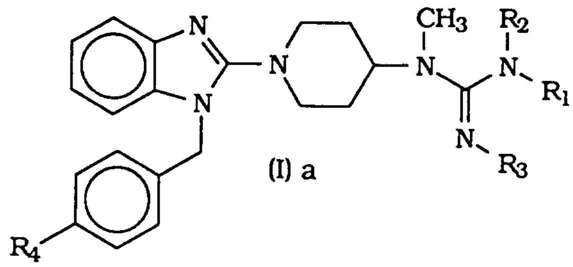

(I) a

erhält, in der $R_1$, $R_2$ und $R_3$ jeweils Wasserstoffatome bedeuten, welche Verbindung (I)a man gewünschtenfalls mit Essigsäureanhydrid umsetzt zur Bildung der Verbindung (I)b

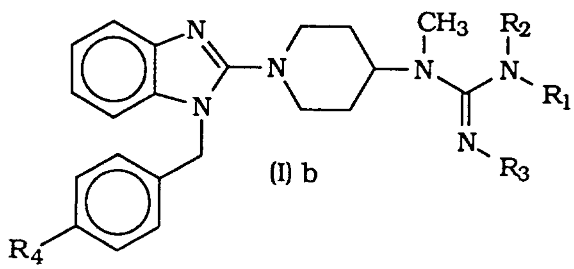

(I) b

worin $R_1$ H, $R_2$ $COCH_3$ und $R_3$ H oder $COCH_3$ bedeuten;
2. oder eine Verbindung (II)

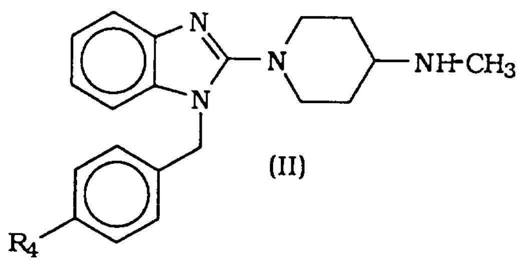

(II)

zunächst mit Kaliumthiocyanat oder mit Methylisothiocyanat und dann mit Methyliodid umsetzt zur Bildung der Verbindung (III)

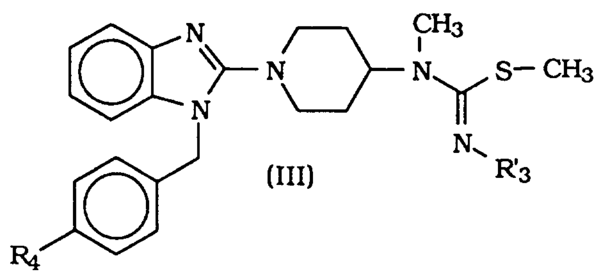

(III)

$R'_3$ = H oder $CH_3$

die man entweder mit Ammoniak oder mit Methylamin oder mit Dimethylamin umsetzt zur Bildung der Verbindung (I)a

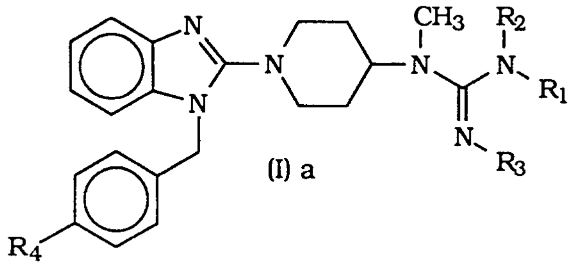

worin $R_1$ H oder $CH_3$, $R_2$ H oder $CH_3$ und $R_3$ H oder $CH_3$ bedeuten, wonach man gewünschtenfalls eine dieser Verbindungen (I)a mit Essigsäureanhydrid umsetzt zur Bildung der Verbindungen (I)b

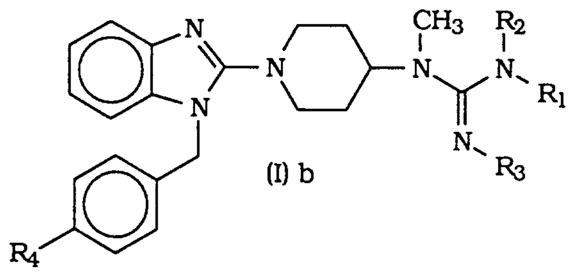

in der entweder $R_1$ $CH_3$, $R_2$ $COCH_3$ und $R_3$ H, $CH_3$ oder $COCH_3$ bedeuten oder $R_1$ $CH_3$, $R_2$ H oder $CH_3$ und $R_3$ $COCH_3$ bedeuten oder $R_1$ H, eine der Gruppen $R_2$ und $R_3$ $CH_3$ und die andere $COCH_3$ bedeuten.

**3.** Verwendung der Verbindungen nach Anspruch 1 als Synthesezwischenprodukte für die Herstellung von Benzimidazol-Derivaten der Formel

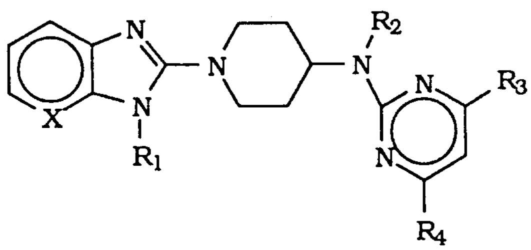

in der
X CH oder N,
$R_1$ entweder ein Wasserstoffatom oder eine Benzylgruppe, die 1 bis 3 Substituenten aufweisen kann, ausgewählt aus Halogenatomen, Trifluormethylgruppen, $(C_{1-4})$-Alkylgruppen, $(C_{1-4})$-Alkoxygruppen, Cyanogruppen, Methylthiogruppen, Methylsulfinylgruppen und Methylsulfonylgruppen, oder eine Heterocyclus-methylgruppe, worin der Heterocyclus ein Pyridinylrest, ein Thienylrest oder ein Furanylrest sein kann, der einen oder mehrere Substituenten aufweisen kann,
$R_2$ ein Wasserstoffatom oder eine $(C_{1-4})$-Alkylgruppe,
$R_3$ ein Wasserstoffatom oder eine Hydroxygruppe,
$R_4$ ein Wasserstoffatom oder eine $(C_{1-4})$-Alkylgruppe bedeuten,
gegebenenfalls in Form der Tautomeren, wenn $R_3$ OH bedeutet, sowie von deren Additionssalzen mit pharmazeutisch annehmbaren Säuren.

**Patentansprüche für folgende Vertragsstaaten : ES, GR**

**1.** Verfahren zur Herstellung von Benzimidazol-Derivaten der Formel (I)

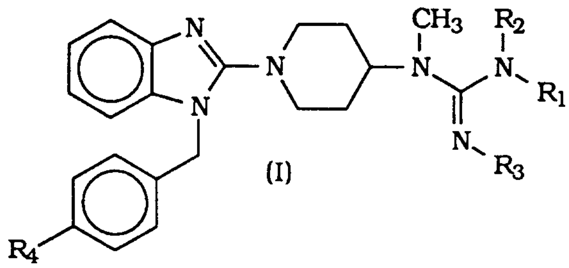

(I)

in der
$R_1$ ein Wasserstoffatom oder eine Methylgruppe.
$R_2$ ein Wasserstoffatom, eine Methylgruppe oder eine Acetylgruppe,
$R_3$ ein Wasserstoffatom, eine Methylgruppe oder eine Acetylgruppe,
$R_4$ ein Wasserstoffatom oder ein Halogenatom
bedeuten sowie deren Additionssalze mit pharmazeutisch annehmbaren Säuren, sowie die tautomeren Formen dieser Verbindungen, **dadurch gekennzeichnet**, daß man

1. entweder eine Verbindung (II)

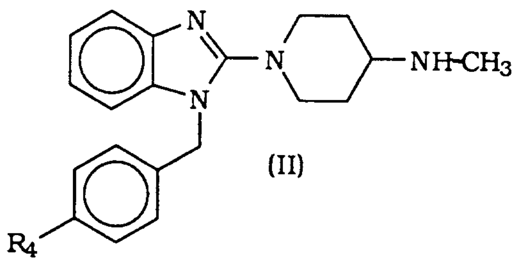

(II)

mit Cyanamid umsetzt, so daß man die Verbindung (I)a

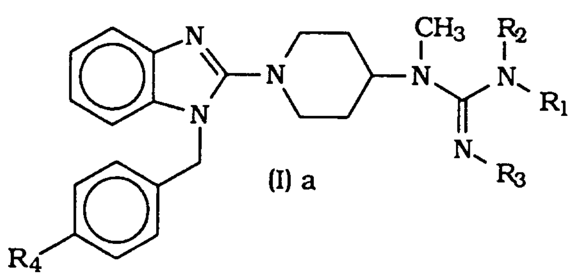

(I) a

erhält, in der $R_1$, $R_2$ und $R_3$ jeweils Wasserstoffatome bedeuten,
welche Verbindung (I)a man gewünschtenfalls mit Essigsäureanhydrid umsetzt zur Bildung der Verbindung (I)b

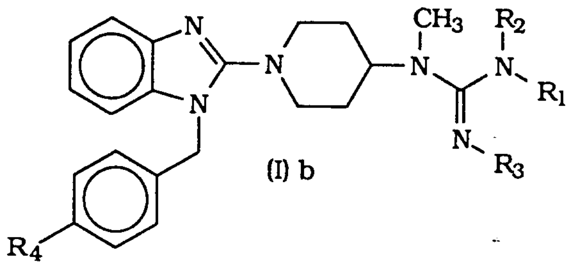

(I) b

worin $R_1$ H, $R_2$ $COCH_3$ und $R_3$ H oder $COCH_3$ bedeuten;
2. oder eine Verbindung (II)

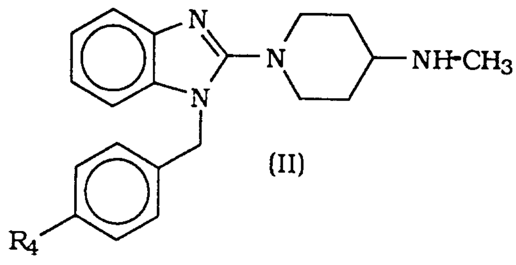

(II)

zunächst mit Kaliumthiocyanat oder mit Methylisothiocyanat und dann mit Methyliodid umsetzt zur Bildung der Verbindung (III)

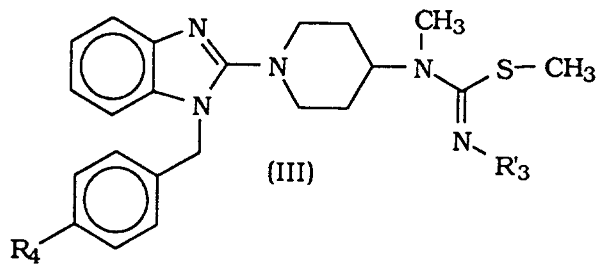

(III)

$R'_3$ = H oder $CH_3$

die man entweder mit Ammoniak oder mit Methylamin oder mit Dimethylamin umsetzt zur Bildung der Verbindung (I)a

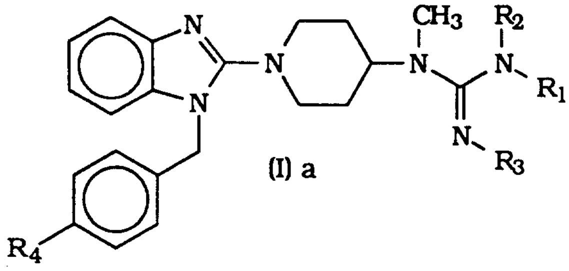

(I) a

worin $R_1$ H oder $CH_3$, $R_2$ H oder $CH_3$ und $R_3$ H oder $CH_3$ bedeuten, wonach man gewünschtenfalls eine dieser Verbindungen (I)a mit Essigsäureanhydrid umsetzt zur Bildung der Verbindungen (I)b

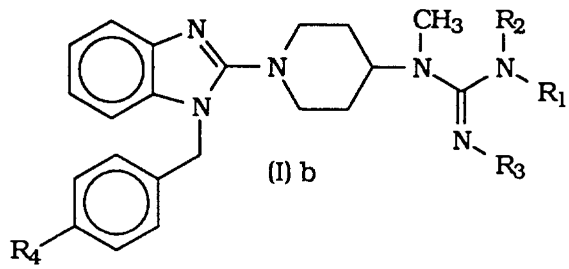

(I) b

in der entweder $R_1$ $CH_3$, $R_2$ $COCH_3$ und $R_3$ H, $CH_3$ oder $COCH_3$ bedeuten oder $R_1$ $CH_3$, $R_2$ H oder $CH_3$ und $R_3$ $COCH_3$ bedeuten oder $R_1$ H, eine der Gruppen $R_2$ und $R_3$ $CH_3$ und die andere $COCH_3$ bedeuten.